# EUROPÄISCHE PATENTSCHRIFT

(11) **EP 0 657 436 B1**
(45) Veröffentlichungstag und Bekanntmachung des Hinweises auf die Patenterteilung: **12.12.2001**
(21) Anmeldenummer: 94810637.2
(22) Anmeldetag: 03.11.1994
(51) Int. Cl.: C07D 221/18, C07D 471/06, C07C 69/76, C07C 233/64, C09B 5/62

(54) **Verfahren zur Herstellung von Perylen-3,4-dicarbonsäurederivaten**
Process for the preparation of perylene-3,4-dicarboxylic acid derivatives
Procédé pour la préparation des dérivés des acides pérylène-3,4-dicarboxyliques

(30) Priorität: 12.11.1993 CH 340193
(43) Veröffentlichungstag der Anmeldung: 14.06.1995
(73) Patentinhaber: Ciba Specialty Chemicals Holding Inc., 4057 Basel (CH)
(72) Erfinder: Langhals, Heinz, Prof. Dr., D-85521 Ottobrunn (DE); Leonhard, Feiler, D-85521 Riemerling (DE)

(56) Entgegenhaltungen:
- EP-A- 0 596 292
- DE-A- 2 512 516
- DE-C- 486 491
- US-A- 3 557 233
- US-A- 4 714 666
- CHEMISCHE BERICHTE, Bd. 124,Nr. 3, März 1991 Seiten 529-535, KAISER; LINDNER; LANGHALS 'Synthese von nichtsymmetrisch substituierten Perylen-Fluoreszenzfarbstoffen'
- BULL. CHEM. SOC. JPN., Bd. 54,Nr. 5, 1981 Seiten 1575-1576, NAGAO; MISONO 'Synthesis and reaction of perylenecarboxylic acid derivatives'
- DYES AND PIGMENTS, Bd. 16, 1991 Seiten 19-25, NAGAO; ABE; MISONO 'Synthesis and properties of N-Alkylbromoperylene-3,4-dicarboximides'
- CHEMICAL ABSTRACTS, vol. 85, no. 3, 19.Juli 1976 Columbus, Ohio, US; abstract no. 20928s, NAGAO; MISONO 'Synthesis and reactions of perylenecarboxylic derivatives' Seite 649; & SHIKIZAI KYOKAISHI, Bd. 49,Nr. 1, 1976 Seiten 29-34,

## Beschreibung

Die vorliegende Erfindung betrifft ein Verfahren zur Herstellung von Perylen-3,4-dicarbonsäurederivaten.

Während die Perylenfarbstoffe, Perylen-3,4:9,10-tetracarbonsäurebisimide (**1**) seit langer Zeit als hoch lichtechte Küpenfarbstoffe und Pigmente, sowie in neuerer Zeit auch als Fluoreszenzfarbstoffe in homogener Lösung Verwendung finden (siehe z.B. H. Zollinger, Color Chemistry, VCH Verlagsgesellschaft, Weinheim, 1987), sind erstaunlicherweise von den Perylen-3,4-dicarbonsäureimiden (**2**) nur wenige, spezielle Vertreter bekannt.

Während für **1** durch die Kondensation des technisch dargestellten Perylen-3,4,9,10-tetracarbonsäurebisanhydrids (**3**) mit primären Aminen ein allgemeiner Syntheseweg zur Verfügung steht, existiert kein präparativer Zugang zu dem analogen Perylen-3,4-dicarbonsäureanhydrid (**4**); **4** ist lediglich über eine Gasphasendecarboxylierung von **3** in verschwindenden Mengen erhalten worden (Z. Iqbal, D.M. Ivory, H. Eckhardt, Mol. Cryst. Liqu. Cryst. 1988, 158b, 337).

Für die Darstellung der wenigen, bekannten N-substituierten Derivate von **2** mußte daher ein Umweg beschritten werden, der allerdings die Auswahl der Substituenten am Imidstickstoff stark einschränkt. Hierfür wurde zunächst das seit 1929 bekannte (DE-PS 486,491), unsubstituierte Perylen-3,4-dicarbonsäureimid **2a** sulfoniert und dann verseift, wobei das sulfonierte Perylen-3,4-dicarbonsäureanhydrid **6** entsteht. Dieses läßt sich mit kurzkettigen (genügend hydrophilen), endständigen, aliphatischen Aminen zu den sulfonierten Dicarbonsäureimiden (**7**) kondensieren. Die Darstellung der entsprechenden Farbstoffe (**2**) erfolgt durch Desulfonierung in halbkonzentrierter Schwefelsäure. Die dafür erforderlichen rauhen Reaktionsbedingungen, die zu Sulfonierungen und Eliminierungen führen können, und die Probleme bei der präparativen Reinigung von sulfonierten Perylen-Derivaten schränken das Verfahren stark ein, so daß bisher nur Substanzen mit den Resten (Wasserstoff) Methyl, Ethyl, 1-Propyl, 1-Butyl, Isobutyl, 1-Pentyl, 1-Hexyl, 1-Octyl, 2-Hydroxyethyl, Phenyl, p-Tolyl und p-Anisyl dargestellt worden sind (Y. Nagao, T. Misono, Chem. Abstr. 85:20928s; Y. Nagao, T. Misono, Bull. Chem. Soc. Japan, 1979, 52, 1723; Y. Nagao, T. Misono, Bull. Chem. Soc. Jpn. 1981, 54, 1575).

Ein Versuch, N-substituierte Perylen-3,4-dicarbonsäureimide mit verschiedenen N-Substituenten, wie z.B. die Verbindung **2c** unten, aus den in der Zwischenzeit präparativ gut zugänglichen Perylen-3,4-dicarbonsäureanhydrid-9,10-dicarbonsäureimiden (H. Kaiser, J. Lindner, H. Langhals, Chem.Ber. 1991, 124, 529) analog zu dem im Bull.Chem. Soc. Jpn. 1981, 54, 1575 für wenige oben erwähnte einfache Imide beschriebenem Verfahren durch Decarboxylierung herzustellen führte nur zu geringen Ausbeuten (max. 4%), so daß dies ebenfalls keinen allgemeinen Syntheseweg zu 2 darstellt.

Bei der Kondensation des Perylen-3,4:9,10-tetracarbonsäurebisanhydrids **3** mit primären Aminen wird üblicherweise in hohen Ausbeuten das entsprechende Perylentetracarbonsäurebisimid **1** erhalten. Führt man aber die Kondensation in Gegenwart von Wasser bei hohen Temperaturen unter Druck und in Gegenwart bestimmter Reagenzien durch, so findet man zum Erstaunen neben den erwarteten Bisimiden **1** auch die Monoimide **2**.

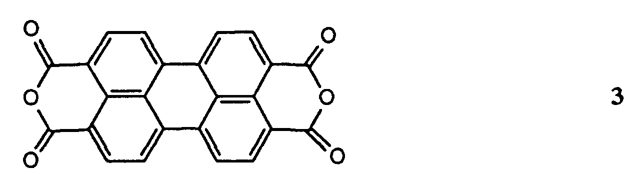

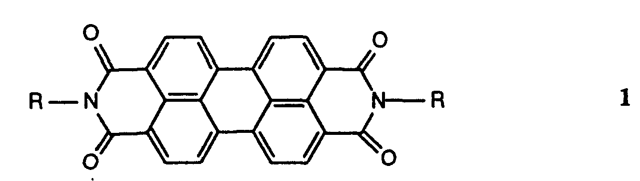

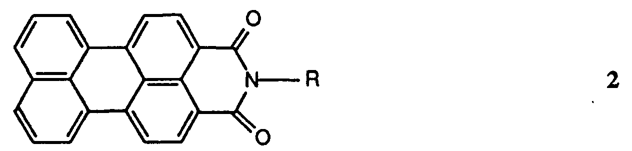

Ein Gegenstand der Erfindung ist daher ein Verfahren zur Herstellung von Perylen-3,4-dicarbonsäureimiden der Formel **I** 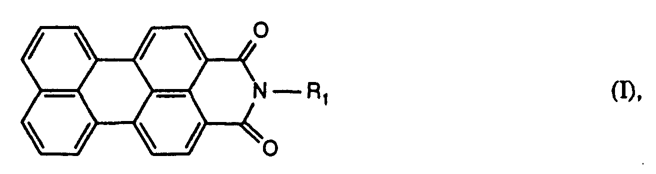 worin R₁ für eine Alkyl-, Aralkyl- oder Cycloalkylgruppe steht oder einen carbocyclischen oder heterocyclischen aromatischen Rest bedeutet, durch Umsetzung des Perylen-3,4,9,10-tetracarbonsäurebisanhydrids mit einem primären Amin R₁-NH₂ bei einer Temperatur von 150-350°C und unter Druck, in Gegenwart von Wasser sowie in Gegenwart eines Zink-, Blei-, Calcium- oder Magnesiumsalzes und eines stickstoffhaltigen Heterocyclus als Base, und wobei das Perylen-Ringsystem nicht substituiert oder einen bis sechs Substituenten tragen kann. Der bevorzugte Temperaturbereich ist bei etwa 180-250°C. Als besonders geeignet hat sich auch eine Reaktionstemperatur von 190°C oder von 210-220°C erwiesen.

Für die Durchführung des erfindungsgemässen Verfahrens besonders geeignete Salze sind Bleiacetat, Zinkchlorid und insbesondere Zinkacetat.

Besonders geeignete stickstoffhaltige Heterocyclen sind Chinolin, Pyridin und insbesondere Imidazol. Vorzugsweise wird die Umsetzung in der jeweiligen heterocyclischen Verbindung als Lösungsmittel durchgeführt.

Die Umsetzung verläuft besonders gut mit primären Aminen, welche einen sterisch gehinderten, vorzugsweise löslichmachenden organischen Rest R₁ aufweisen. Als besonders geeignete Reste R, haben sich insbesondere die folgenden Reste erwiesen: 2,5-Di-tert-butylphenyl, 2,5-Di-tert-butyl-4-nitrophenyl, 4-tert-Butylphenyl, 2,3-Dimethylphenyl, 1-Hexylheptyl, 1-Octylnonyl, 1-Nonyldecyl, Cyclopropyl, Cyclopentyl, Cyclohexyl, Cycloheptyl, Cyclooctyl, Cyclododecyl, Adamantyl oder 4-Carbamoylphenyl.

Die optimale Menge des für die erfindungsgemässe Umsetzung zugesetzten Wassers, des primären Amins R₁-NH₂, oder des verwendeten Salzes kann von Fall zu Fall variieren. Bei der Herstellung des Monoimids **2b** (R₁ gleich 2,5-Di-tert-butylphenyl) aus dem Bisanhydrid **3** kann man geeigneterweise etwa 0.7 ml Wasser/mmol **3,** etwa 1.6 mol 2,5-Di-tert-butylanilin/mol **3** und etwa 135 mg Zinkacetatlmmol **3** einsetzen.

Unter diesen Bedingungen erhält man die Verbindung **2b** in einer isolierten Ausbeute von etwa 50%. Der Rest ist im wesentlichen nicht umgesetztes Ausgangsmaterial, Perylen und das entsprechende Bisimid **1b,** welche leicht chromatographisch abgetrennt werden können. Die optimale Reaktionstemperatur ist etwa 190°C. Bei höheren Temperaturen werden größere Anteile an Perylen erhalten, und bei niedrigeren Temperaturen sinkt die Ausbeute. Für die Aufarbeitung kann es günstig sein, die Reaktion bei der höheren Temperatur von 210-220°C und bei einer kürzeren Reaktionszeit von etwa 7-8 h durchzuführen. Die Ausbeute ist dann zwar etwas geringer, und es wird auch mehr Perylen gebildet, dieses kann aber sehr leicht chromatographisch abgetrennt werden. Dagegen entsteht dann kaum das entsprechende Bisimid **1b**, dessen Abtrennung etwas schwieriger ist.

Mit einer Ausbeute von 50% bei der Umsetzung im Autoklaven ist das Monoimid **2b** ein geeignetes Ausgangsmaterial für die Darstellung des Perylen-3,4-dicarbonsäureanhydrids **4.** Hierfür wird **2b** beispielsweise mit KOH in tert-Butylalkohol umgesetzt, und es erfolgt die Verseifung zum Monoamid der Perylen-3,4-dicarbonsäure. Wird die alkalische Lösung des so erhaltenen Monoamids angesäuert, so erfolgt zum Teil eine weitere Verseifung, wobei das gewünschte Perylen-3,4-dicarbonsäureanhydrid **(4)** gebildet wird (zum Teil erfolgt eine Rückreaktion zum Ausgangsmaterial **2b**). Als besonders geeignete Säure hat sich dabei 50 proz. Essigsäure erwiesen, wobei Ausbeuten an **4** von über 60% erreicht werden; auf den Umsatz bezogen beträgt die isolierte Ausbeute an **4** sogar über 90%. Der restliche Anteil an Cyclisierungsprodukt **2b** läßt sich dabei problemlos durch Lösen mit heißer Kaliumcarbonat-Lösung, Filtrieren und Ausfällen mit Essigsäure abtrennen, so daß das Anhydrid **4** in hoher Reinheit erhalten wird.

Das so erhaltene Anhydrid **4** kann mit beliebigen primären Aminen zu den Perylen-3,4-dicarbonsäureimiden **(2)** unter den oben beschriebenen Reaktionsbedingungen, z.B. mit Zinkacetat in Imidazol oder Chinolin, kondensiert werden.

Ein weiterer Gegenstand der Erfindung ist somit auch ein Verfahren zur Herstellung von Perylen-3,4-dicarbonsäureimiden der oben angegebenen Formel I durch Umsetzung des Perylen-3,4-dicarbonsäureanhydrids mit einem primären Amin R₁-NH₂ bei einer Temperatur von 150-350°C und unter Druck in Gegenwart eines Zink-, Blei-, Calcium- oder Mangansalzes und eines stickstoffhaltigen Heterocyclus als Base.

Aus den oben beschriebenen und nun gut zugänglichen Perylen-3,4-dicarbonsäureimiden **(2)** und Perylen-3,4-dicarbonsäureanhydrid **(4)** lässt sich problemlos durch Anwendung allgemein bekannter Umsetzungen eine Vielzahl von Derivaten der Perylen-3,4-dicarbonsäure herstellen.

Bedeutet der oben definierte Rest R₁ Alkyl, so handelt es sich vorzugsweise um C₁-C₄₁-Alkyl. Die Reste können geradkettig oder verzweigt sein. Bevorzugt sind sekundäre Alkylreste, wie z.B. 1-Hexylheptyl, 1-Heptyloctyl, 1-Octylnonyl oder 1-Nonyldecyl.

Aralkyl bedeutet z.B. Benzyl.

Cycloalkyl kann mono- oder auch polycyclisch sein mit vorzugsweise 3-12 Kohlenstoffatomen im Ring. Beispiele geeigneter Reste sind Cyclopropyl, Cyclobutyl, Cyclopentyl, Cyclohexyl, Cyclooctyl, Cyclododecyl, Decalinyl oder Adamantyl.

Carbocyclische oder heterocyclische aromatische Reste können ebenfalls einen oder mehrere, gegebenenfalls kondensierte Ringe aufweisen, welche vorzugsweise fünf- oder sechsgliedrig sind. Die heterocyclischen aromatischen Reste haben vorzugsweise ein, zwei oder drei Heteroatome, insbesondere N-, O-, oder S-Atome im Ring. Die carbocyclischen aromatischen Reste weisen vorzugsweise 6-12 C-Atome auf. Beispiele geeigneter Reste sind Phenyl, Tolyl, Naphthyl oder Biphenyl. Geeignete heterocyclische aromatische Reste sind z.B. Furyl, Thienyl, Pyrrolyl, Imidazolyl, Pyrazolyl, Isothiazolyl, Isoxazolyl, Pyridyl, Pyrazinyl,Pyrimidinyl, Pyridazinyl, Indolyl, Isoindolyl, Indazolyl, Chinolyl, Isochinolyl, Quinazolinyl oder Carbazolyl.

Die erfindungsgemässen Perylen-3,4-dicarbonsäurederivate der Formel I sind vorzugsweise im Perylen-Ringsystem nicht substituiert. Sie können aber auch einen oder mehrere, in der Regel jedoch höchstens sechs Substituenten im Ringsystem aufweisen, wobei die Substituenten unabhängig voneinander Alkyl, Aralkyl, Cycloalkyl, Alkoxy, Aryloxy, Alkylaryl, Alkylmercapto, Arylmercapto, einen carbocyclischen oder heterocyclischen aromatischen Rest oder Chloro, Bromo, Nitro, -SO₃H (sowie deren Metallsalze oder deren Ammoniumsalze) oder -SO₃R (wobei R für Alkyl oder Aryl steht), Amino, Acylaminomethyl, wie z.B. Acetylaminomethyl, Alkylamino, Arylamino, Phthalimidomethyl, Aminomethyl, Dimethylaminomethyl (hergestellt zum Beispiel durch Spaltung des entsprechenden Phthalimido-Derivats), Pyrazolomethyl, sein können.

Die letztgenannten sulfo- bzw. amino-substituierten Perylen-3,4-dicarbonsäurederivate eignen sich insbesondere als Rheologieverbesserer. Entsprechende Derivate anderer Pigmentsysteme, wie z.B. der Phthalocyaninpigmente oder der Quinacridonpigmente sowie deren Herstellung sind beispielsweise aus US 4,981,888, EP-A 356,390, EP-A 508,704, US 5,212,221 oder EP-A 485,337 bekannt. Die vorliegenden substituierten Perylen-3,4-dicarbonsäurederivate können auf analoge Weise hergestellt werden.

Vorzugsweise sind der Substituent bzw. die Substituenten in 1-, 9-, bzw. 1,6-, 1,9-, 2,5-, 7,12-, 8,11- oder 9,10-Stellung. Die substituierten Perylenderivate weisen vorzugsweise einen oder zwei Substituenten im Ringsystem auf, und bei disubstituierten Verbindungen sind die Substituenten vorzugsweise gleich.

Die substituierten Perylenderivate können aus den entsprechenden nicht substituierten Verbindungen nach allgemein bekannten Methoden hergestellt werden oder auch durch Umwandlung eines bereits substituierten anderen Derivates (z.B. substituierte Diester aus substituierten Imiden) synthetisiert werden. Als Beispiel wird im Folgenden die Herstellung der Nitro-, Amino- und Bromo-Derivate beschrieben.

Die Perylendicarbonsäureimide **(2)** können z.B. mit verschiedenen Reagenzien nitriert werden. Eine Umsetzung von **2b** (R₁ gleich 2,5-Di-tert-butylphenyl) mit Salpetersäure in Eisessig führt zu einer Vielzahl an Produkten, von denen das 1,6-Dinitro-Derivat chromatographisch problemlos mit einer Ausbeute von 13% isoliert werden kann.

Bei einer Reaktion von **2c** (R₁ gleich 1-Hexylheptyl) mit Salpetersäure in Acetanhydrid lassen sich chromatographisch 8% des 1-Nitro-Derivats, 35% des 1,9-Dinitro-Derivats und 14% des 9,10-Dinitro-Derivats isolieren.

Eine Nitrierung von **2c** mit N₂O₄ in Methylenchlorid erzeugt ein komplexes Reaktionsgemisch, aus dem 7% der 1-Nitro-Verbindung chromatographisch abgetrennt werden kann. Eine Katalyse der Reaktion durch Methansulfonsäure fördert eine Zweitsubstitution. Es werden dann 12% 1,6-Dinitro-, 36% 9-Nitro-, 9% 2,5-Dinitro- und 25% 9,10-Dinitro-Substitutionsprodukt gefunden.

Die Nitrierung von **2c** durch N₂O₄ in Methylenchlorid wird stark durch Licht beeinflußt. Wegen der breiten Lichtabsorption von **2c** im sichtbaren Bereich reicht hierfür bereits normales Tageslicht völlig aus, und die Photoreaktionen laufen mit hohen Quantenausbeuten ab.

Wird die Nitrierung von **2c** mit N₂O₄ in Methylenchlorid bei völliger Dunkelheit durchgeführt, so lassen sich als Reaktionsprodukt 55% des 9-Nitro-Derivats als Reinsubstanz isolieren. Der Rest ist fast ausschließlich nicht umgesetztes Ausgangsmaterial, das leicht abgetrennt werden kann. Diese Reaktion ist daher der beste Weg, das 9-Nitro-Derivat in präparativem Maßstab herzustellen.

Die 9-Nitroverbindung läßt sich mit Eisen in Eisessig zum 9-Amin reduzieren, von dem direkt 30% erhalten werden. Durch eine chromatographische Aufarbeitung der Rückstände lassen sich weitere 20% isolieren. Bessere Resultate werden bei der Reduktion mit Eisen in Salzsäure erzielt. Hiermit läßt sich das Amin direkt in 85% Ausbeute isolieren.

Das oben erwähnte 9-Amin ist im Gegensatz zu den Perylenfarbstoffen 1 bemerkenswert stark positiv solvatochrom. Sein Absorptionsmaximum wird von 554 nm in Chloroform bis zu 602 nm in Methanol verschoben. Da der Farbstoff verhältnismäßig lichtecht ist und außerdem schwach fluoresziert, ist er für die Erzeugung der dritten harmonischen Welle in einer nichtlinearen Optik über einen Resonanz-Effekt von Interesse.

Eine Derivatisierung von **2c** mit Brom (vgl. auch Y. Nagao, Y. Abe, T. Misono, Dyes. Pigm. 1991, 16, 19) in Chlorbenzol liefert in 63% Ausbeute das 9-Brom-Derivat neben mehrfach bromierten Produkten. Die Reaktion entspricht damit der Nitrierung unter Lichtausschluß. **2b** läßt sich analog bromieren. Es entstehen bei der Reaktion jedoch auch polybromierte **2b,** die nicht abzutrennen waren. Wird die Bromierung dagegen in Chlorbenzol in Gegenwart von Kaliumcarbonat ausgeführt, so kann man 77% 9-Brom-2b isolieren.

Die erfindungsgemässen Perylen-3,4-dicarbonsäurederivate der Formel I fluoreszieren in Lösung sehr stark und mit hohen Quantenausbeuten. An Lichtechtheit übertreffen sie sogar die als außerordentlich photostabil bekannten Perylenfarbstoffe (Perylenbisimide **1,** oben), welche die zur Zeit stabilsten Fluoreszenzfarbstoffe überhaupt sind. So bleicht z.B. das Monoimid **2b** in Dimethylformamid-Lösung um einen Faktor 20 langsamer als **1b** aus. Ähnliche Ergebnisse werden für die anderen Farbstoffe **2** gefunden. Die Löslichkeit der Farbstoffe ist im allgemeinen höher als die der entsprechenden Perylenfarbstoffe **1**. Die verhältnismäßig schmale Absorptions- und Fluoreszenzbande der erfindungsgemässen Verbindungen führt zu brillanten Farbtönen. Günstig für eine große Farbstärke ist die nahezu rechteckförmige Absorptionsbande.

Als Feststoffe bilden die erfindungsgemässen Verbindungen leuchtend rote Pigmente, welche überraschenderweise auch als Feststoffe intensiv fluoreszieren. Sie sind daher auch als lichtechte Fluoreszenzpigmente interessant. Bei wohlgeordneten Kristalliten wird in der Regel jeweils eine intensive, langwellige Fluoreszenzbande erhalten. Wird dagegen die Substanz sehr fein pulverisiert oder schnell aus einer Lösung gefällt, so wird die kürzerwellige Fluoreszenzbande zur intensitätsstärksten, und das Feststoffspektrum ähnelt dann mehr den Fluoreszenzspektrum von Lösungen, ist demgegenüber aber leicht hypsochrom verschoben.

Die erfindungsgemässen Verbindungen eignen sich anhand ihrer Eigenschaften für eine Vielzahl von Anwendungen.

So können sie beispielsweise als Pigmente für die Massefärbung von Kunststoffen oder Lacken eingesetzt werden.

Geeignete Kunststoffe sind z.B. Polyolefine, Polyvinylchlorid, Fluorpolymerisate, wie z.B. Polyfluorethylen, Polytrifluorchlorethylen oder Tetrafluorethylen/Hexafluorpropylen-Mischpolymerisat, Silikonharze, insbesondere aber Ingenieurwerkstoffe (Engineering Plastics), wie z.B. Polycarbonate, Polyacrylate, Polymethacrylate, Polystyrol, ABS, Polyester, insbesondere Polyalkylenterephthalate, wie Polybutylenterephthalat (PBT) oder Polyethylenterephthalat (PET), Polyamide, Polyetherketone, Polyurethane, einzeln oder in Mischungen. Zweckmässig werden die erfindungsgemässen Verbindungen in einer Konzentration von 0.01 bis 10, vorzugsweise 0.01-5 Gew.%, bezogen auf das Polymere, eingesetzt.

Als Beispiele für Polyolefine, die mit den erfindungsgemässen Verbindungen gefärbt werden können, seien Polyethylen hoher und niederer Dichte (HD-PE, LD-PE und LLD-PE), Polyisobutylen und insbesondere Polypropylen, sowie Copolymere von Polyolefinen mit z.B. Polyethem, Polyetherketonen oder Polyurethanen, erwähnt. Bevorzugt ist Polypropylen.

Die Färbung erfolgt nach den üblichen Verfahren, beispielsweise durch Mischen einer erfindungsgemässen Verbindung oder eines Gemisches solcher Verbindungen mit dem Kunststoffgranulat oder -pulver, ohne es vorher in ein Präparat einarbeiten zu müssen, und Extrudieren der Mischung zu Fasern, Folien oder Granulaten. Letztere können dann beispielsweise im Spritzgussverfahren zu Gegenständen verformt werden.

Die erhaltenen rot fluoreszierenden Ausfärbungen weisen hohe Reinheit und hohe Sättigung auf und zeichnen sich durch gute Transparenz sowie durch gute Beständigkeit, insbesondere gegen Licht aus.

Erfindungsgemässe Verbindungen, welche einen oder mehrere Substituenten ausgewählt aus der Gruppe -SO₃H (sowie deren Metallsalze oder deren Ammoniumsalze) oder -SO₃R (wobei R für Alkyl oder Aryl steht), Amino, Acylaminomethyl, wie z.B. Acetylaminomethyl, Alkylamino, Arylamino, Phthalimidomethyl, Aminomethyl, Dimethylaminomethyl (hergestellt zum Beispiel durch Spaltung des entsprechenden Phthtalimido-Derivats) oder Pyrazolomethyl aufweisen, können zudem als Rheologieverbesserer eingesetzt werden.

Die nachfolgenden Beispiele erläutern die Erfindung.

### Beispiele 1-4: Herstellung von Perylendicarboximiden aus Perylentetracarbonsäurebisanhydrid im Autoklav

### Beispiel 1: N-(2,5-Di-t-butylphenyl)-perylen-3,4-dicarboximid (2b)

In einem 100 ml fassenden Autoklav wird eine Mischung aus 3.66 g (9.34 mmol) Perylen-3,4:9,10-tetracarbonsäurebisanhydrid mit 18.7 g Imidazol, 1.32 g Zinkacetatdihydrat, 8.0 ml (450 mmol) Wasser und 1.05 g (5.12 mmol) 2,5-Di-tert-butylanilin (hergestellt gemäss Rec. Trav. Chim. Pays-Bas, 1958, 77, 491) 23 h auf 210°C erhitzt. Nach Reaktionsende wird die Mischung mit Ethanol aus dem Autoklaven gespült, mit Wasser und konz. Salzsäure versetzt und solange gekocht, bis aller Ethanol verdampft ist. Man saugt den braunroten Rückstand ab und kocht ihn mit 10 proz. Kaliumcarbonatlösung 1 h lang. Der Rückstand wird abgesaugt, im Trockenschrank bei 120°C getrocknet und mit Chloroform über Kieselgel chromatographiert. Man erhält hierbei Perylen als Vorlauf (gelb, blau fluoreszierend) gefolgt von N-(2,5-Di-tert-butylphenyl)-perylen-3,4-dicarboximid (**2b**) und dann N,N'-Bis(2,5-Di-tert-butylphenyl)-perylen-3,4,9,10-tetracarboximid (**1b**). Ausb. 2.40 g (50.5%), Schmp. >300°C. R_{f} (CHCl₃/Silikagel)= 0.85. UV (CHCl₃): λₘₐₓ (ε)= 489 nm (35300), 512 (33590). Fluoreszenz (CHCl₃, Anr. 489 nm) λₘₐₓ (Iᵣₑₗ)= 535 nm (1), 576nm (0.36).

| | | | | |
|---|---|---|---|---|
| C₃₆H₃₁NO₂ (509.7) | Ber. | C 84.84 | H 6.13 | N 2.75 |
| | Gef. | C 84.79 | H 6.35 | N 2.81 |

### Beispiel 2: N-(4-t-Butylphenyl)-perylen-3,4-carboximid aus Perylen-3,4,9,10-tetracarbonsäurebisanhydrid

Man mischt 3.66 g (9.33 mmol) Perylen-3,4,9,10-tetracarbonsäurebisanhydrid mit 18.66 g Imidazol, 1.36 g Zinkacetatdihydrat, 8 ml Wasser und 1.89 g (12.7 mmol) 4-t-Butylanilin und erhitzt 10 h lang im Autoklav auf 220°C. Das Reaktionsprodukt wird mit Ethanol aus dem Autoklav herausgewaschen, mit 200 ml 10% Salzsäure versetzt und so lange gekocht, bis kein Ethanol mehr enthalten ist. Der Niederschlag wird abgesaugt und dann mit 10% Kaliumcarbonatlösung aufgekocht. Der unlösliche Rückstand wird abgesaugt, mit Wasser gewaschen und im Trockenschrank bei 120°C getrocknet. Durch Chromatographie mit Chloroform über Kieselgel wurden 2 Produkte mit den R_{f}-Werten 0.40 und 0.19 isoliert, die durch ¹H-NMR-Spektroskopie als N-(4-t-Butylphenyl)-perylen-3,4-carboximid und N,N'-Di(4-t-butylphenyl)-perylen-3,4,9,10-biscarboximid identifiziert werden können. Wegen der schlechten Löslichkeit der Produkte in Chloroform wird nur ein kleiner Teil des Rohproduks chromatographisch gereinigt und dann die erhaltenen Mengen auf die Gesammtausbeute hochgerechnet. Man erhielt so etwa 10% N-(4-t-Butylphenyl)-perylen-3,4-carboximid und etwa 30% N,N'-Di(4-t-butylphenyl)-perylen-3,4,9,10-biscarboximid.

### Beispiel 3: N-1-Hexylheptyl-perylen-3,4-dicarboximid (2c)

1.2 g (3.1 mmol) Perylen-3,4:9,10-tetracarbonsäurebisanhydrid, 6.2 g Imidazol, 470 mg (2.36 mmol) 7-Aminotridecan (hergestellt gemäss J. Prakt. Chem. 1980, 322, 261), 150 mg (0.68 mmol) Zinkacetatdihydrat und 3.0 ml (170 mmol) Wasser werden in einem Autoklaven (100 ml) 24 h auf 190°C erhitzt. Die Aufarbeitung erfolgt analog zur Darstellung von 2b. Ausb. 1.25 g (27%).

### Beispiel 4: N-1-Octylnonyl-perylen-3,4-dicarboximid (2d)

1.4 g (3.1 mmol) Perylen-3,4:9,10-tetracarbonsäurebisanhydrid, 6.1 g Imidazol, 960 mg (3.7 mmol) 9-Aminoheptadecan (hergestellt gemäss DE-A 4,007,618), 150 mg (0.68 mmol) Zinkacetatdihydrat und 3.0 ml (170 mmol) Wasser werden in einem Autoklaven (100 ml) 24 h auf 190°C erhitzt. Die Aufarbeitung erfolgt analog zur Darstellung von 2b. Ausb. 130 mg (7% extraktiv umkristallisiert aus Methanol, wie in Chem. Ber. 1985, 118, 4641 beschrieben) orangerote Kristalle mit starker Feststofffluoreszenz, Schmp. 143-143.6°C. Rf (CHCl₃/Kieselgel)= 0.94. UV (CHCl₃): λₘₐₓ (ε)= 454 nm (sh, 17620), 485 (32530), 508 (29480). Fluoreszenz (CHCl₃, Anr. 485 nm): λₘₐₓ (Iᵣₑₗ)= 572 (sh, 0.49), 536 (1).

| | | | | |
|---|---|---|---|---|
| C₃₉H₄₅NO₂ (559.8) | Ber. | C 83.68 | H 8.10 | N 2.50 |
| | Gef. | C 83.62 | H 8.02 | N 2.73 |

### Beispiele 5-13: Herstellung von Perylendicarboximiden aus Perylendicarbonsäureanhydrid

### Beispiel 5: N-1-Hexylheptyl-perylen-3,4-dicarboximid (2c)

200 mg (620 mmol) Perylen-3,4-dicarbonsäureanhydrid (hergestellt gemäss Beispiel 20, unten) werden mit 250 mg (0.12 mmol) 7-Aminotridecan, 110 mg Zinkacetatdihydrat und 1.2 g Imidazol unter Ar-Schutzatmosphäre 1 h auf 135-140°C erhitzt. Nach dem Erkalten wird der dunkelrote Schmelzkuchen mit 100 ml Ethanol digeriert und die entstandene Suspension mit 100 ml 15 proz. Salzsäure versetzt und so lange gekocht, bis aller Ethanol verdampft ist (das Reaktionsprodukt ist in Ethanol leicht löslich). Der Feststoff wird abgesaugt und so lange mit warmem Wasser gewaschen, bis das ablaufende Filtrat nicht mehr gelb fluoresziert. Das Rohprodukt wird anschließend 2 h im Trockenschrank bei 120°C getrocknet und anschließend mit Chloroform über Kieselgel chromatographiert. Die erste farbige Fraktion wird aus Methanol extraktiv umkristallisiert. Ausb. 200 mg (63%) orangerote Kristalle mit starker Feststofffluoreszenz, Schmp. 166-168°C. R_{f} (Chloroform/Kieselgel)= 0.87. UV (CHCl₃): λₘₐₓ (ε)= 506 (30250), 484 (31580). Fluoreszenz (CHCl₃, Anr. 506 nm) λₘₐₓ (Iᵣₑₗ): 540 (1), 568 (0.52).

| | | | | |
|---|---|---|---|---|
| C₃₅H₂₇NO₂ (503.7) | Ber. | C 83.50 | H 7.36 | N 2.78 |
| | Gef. | C 83.58 | H 7.28 | N 2.92 |

### Beispiel 6: N-1-Nonyldecyl-perylen-3,4-dicarboximid (2e)

230 mg (0.70 mmol) Perylen-3,4-dicarbonsäureanhydrid werden mit 400 mg (1.40 mmol) 10-Aminononadecan (hergestellt gemäss der DE-A 4,007,618) und 1.0 g Imidazol unter Ar-Schutzatmosphäre wie bei 2c umgesetzt und aufgearbeitet. Nach der Chromatographie mit Chloroform an Kieselgel wird das Reaktionsprodukt mit Petrolether auf Kieselgel aufgezogen, und die rein aliphatischen Nebenprodukte mit ca. 1 1 Petrolether ausgewaschen. Das Reaktionsprodukt wird dann mit Toluol eluiert und anschließend aus Pentan extraktiv umkristallisiert. Ausb. 400 mg (85%), Smp. 143-143.5°C. R_{f} (Kieselgel/CHCl₃)= 0.94. UV (CHCl₃): λₘₐₓ (ε)= 507nm (31319), 482 (32213), 453 (sh, 17450). Fluoreszenz (CHCl₃, Anr. 507 nm) λₘₐₓ (Iᵣₑₗ)= 539 (1), 557 (0.47).

| | | | | |
|---|---|---|---|---|
| C₄₁H₄₉NO₂ (587.8) | Ber. | C 83.77 | H 8.40 | N 2.38 |
| | Gef. | C 84.03 | H 8.47 | N 2.52 |

### Beispiel 7: N-Cyclooctyl-perylen-3,4-dicarboximid (2f)

300 mg (0.93 mmol) Perylen-3,4-dicarbonsäureanhydrid werden mit 1.5 g (2.15 mmol) Cyclooctylamin und 3 g Imidazol unter Ar-Schutzatmosphäre wie bei 2c 12 h bei 140°C umgesetzt und aufgearbeitet. Nach dem Trocknen im Trockenschrank wird zum Entfernen von nicht umgesetztem Ausgangsmaterial mit 10 proz. Kaliumcarbonatlösung aufgekocht und so lange mit heißem Wasser ausgewaschen, bis das Filtrat farblos abläuft. Das braunrote Produkt wird bei 120°C im Trockenschrank getrocknet und dann extraktiv aus Ethanol umkristallisiert. Ausb. 290 mg (72%) dunkelrotes Kristallpulver ohne Feststofffluoreszenz, Schmp. 342-343°C, R_{f} (CHCl₃/Kieselgel)= 0.78. UV (CHCl₃): λₘₐₓ (ε)= 506 nm (32000), 484 (32100), 264 (35200). Fluoreszenz (CHCl₃) λₘₐₓ (Iᵣₑₗ)= 511 nm, 542 (1), 573 (sh, 0.51).

| | | | | |
|---|---|---|---|---|
| C₃₀H₂₅NO₂ (431.5) | Ber. | C 83.50 | H 5.84 | N 3.25 |
| | Gef. | C 83.22 | H 5.83 | N 3.53 |

### Beispiel 8: N-Cyclododecyl-perylen-3,4-dicarboximid (2g)

190 mg (0.56 mmol) Perylen-3,4-dicarbonsäureanhydrid werden mit 140 mg (1.28 mmol) Cyclododecylamin und 2.0 g Imidazol unter Ar-Schutzatmosphäre wie bei 2c umgesetzt und aufgearbeitet. Nach der Chromatographie mit Chloroform an Kieselgel wird das Reaktionsprodukt mit Toluol extraktiv umkristallisiert. Ausb. 190 mg (66%), Schmp. 285°C, R_{f} (CHCl₃/Kieselgel)= 0.58. UV (CHCl₃): λₘₐₓ (ε): 454 (Schulter, 19085), 484 (32630), 507 (32015). Fluoreszenz (CHCl₃, Anr. 584 nm) λₘₐₓ (Iᵣₑₗ)= 538 (1), 572 (sh, 0.44).

| | | | | |
|---|---|---|---|---|
| C₃₄H₃₃NO₂ (487.6) | Ber. | C 83.75 | H 6.82 | N 2.87 |
| | Gef. | C 83.75 | H 6.81 | N 2.91 |

### Beispiel 9: N-2-Hydroxyethyl-perylen-3,4-dicarboximid (2h)

190 mg (0.56 mmol) Perylen-3,4-dicarbonsäureanhydrid werden mit 0.85 ml (1.40 mmol) 2-Aminoethanol und 1.18 g Imidazol unter Ar-Schutzatmosphäre wie bei 2c 96 h umgesetzt und aufgearbeitet. Nach dem Trocknen im Trockenschrank wird zum Entfernen von nicht umgesetztem Ausgangsmaterial mit 10 proz. Kaliumcarbonatlösung aufgekocht und so lange mit heißem Wasser ausgewaschen, bis das Filtrat farblos abläuft. Das braunrote Produkt wird bei 120°C im Trockenschrank getrocknet und dann extraktiv aus Toluol umkristallisiert. Ausb. 50 mg (23%), Schmp. >260°C. UV (CHCl₃): λₘₐₓ= 489nm, 509. Fluoreszenz (CHCl₃, Anr. 489 nm) λₘₐₓ (Iᵣₑₗ)= 546nm (1), 579 (sh, 0.46).

| | | | | |
|---|---|---|---|---|
| C₂₄H₁₅NO₃ (365.4) | Ber. | C 78.89 | H 4.14 | N 3.83 |
| | Gef. | C 78.26 | H 4.27 | N 3.84 |

### Beispiel 10: N-(4-t-Butylphenyl)-perylen-3,4-dicarboximid

Man mischt 0.70 g (2.17 mmol) Perylen-3,4-dicarbonsäureanhydrid mit 0.49 g (3.28 mmol) 4-t-Butylanilin, 3.00 g Imidazol und 0.10 g Zinkacetatdihydrat und erhitzt die Mischung 5 h lang auf 140-150°C. Noch heiss versetzt man die Mischung mit 100 ml Ethanol und gibt dann noch 200 ml 10% Salzsäure zu. Die rote Suspension wird so lange erhitzt, bis aller Ethanol abgedampft ist, dann saugt man ab. Der rotbraune Rückstand wird mit 10% Kaliumcarbonatlösung ausgekocht, dann saugt man heiss ab und spült einige Male mit heissem dest. Wasser nach. Der rote Rückstand wird im Trockenschrank bei 120°C getrocknet und anschliessend mit Toluol/Methanol-Mischung extraktiv umkristallisiert. Man erhält so 0.80 g (81%) rotes N-(4-t-Butylphenyl)-perylen-3,4-dicarboximid, das eine rote Festkörperfluoreszenz zeigt. Smp. >330°C. R_{f}(CHCl₃/Kieselgel)= 0.35.

| | | | | |
|---|---|---|---|---|
| C₃₂H₂₃NO₂ (453.5) | Ber. | C 84.74 | H 5.11 | N 3.09 |
| | Gef. | C 84.58 | H 5.05 | N 3.16 |

### Beispiel 11: N-Cyclopentyl-perylen-3,4-dicarboximid

Man mischt 0.30g (0.93 mmol) Perylen-3,4-dicarbonsäureanhydrid mit 0.24 (2.80mmol) Cyclopentylamin und 3 g Imidazol und erhitzt die Mischung unter Stickstoff 4 h lang auf 145°C. Das Reaktionsprodukt wird mit Ethanol aus dem Kolben gewaschen, dann mit 100 ml 10% Salzsäure versetzt und so lange gekocht, dass kein Ethanol mehr enthalten ist. Das ausgefallene Produkt wird abgesaugt, mit Kaliumcarbonatlösung ausgekocht, abgesaugt und mit heissem dest. Wasser gewaschen. Durch Ansäuern des Filtrats mit konz. Salzsäure kann 60 mg (20%) Perylen-3,4-dicarbonsäureanhydrid zurückgewonnen werden. Der rote Rückstand wird 3 mal mit Methanol extraktiv umkristallisiert und dann im Trockenschrank bei 120°C getrocknet. Ausb. 0.22 g (61%) dunkelrote Nädelchen mit starker roter Festkörperfluoreszenz, Smp. >315°C. R_{f} (CHCl₃/Kieselgel)= 0.18. UV (CHCl₃): λₘₐₓ (ε)= 506 (31013), 485 (31425), 355 (3050), 336 (3000), 319 (2680), 264 (34268), 256 (Sh., 18100). Fluoreszenz (CHCl₃): λₘₐₓ (Iᵣₑₗ)= 541(1), 572 (Sh., 0.49).

| | | | | |
|---|---|---|---|---|
| C₂₇H₁₉NO₂ (389.5) | Ber. | C 83.27 | H 4.92 | N 3.60 |
| | Gef. | C 82.45 | H 4.69 | N 3.45 |

### Beispiel 12: N-Cyclohexyl-perylen-3,4-dicarboximid

Man mischt 0.30 g (0.93 mmol) Perylen-3,4-dicarbonsäureanhydrid mit 0.28 g (2.8 mmol) Cyclohexylamin und 3 g Imidazol und erhitzt unter Stickstoff 3h lang auf 140°C. Das Reaktionsprodukt wird mit Ethanol aus dem Kolben gewaschen, mit 200 ml 10% Salzsäure versetzt und so lange gekocht, bis kein Ethanol mehr enthalten ist Der Rückstand wird abgesaugt, mit Kaliumcarbonatlösung gekocht und wiederum abgesaugt. Der Rückstand wird mit heissem Wasser gewaschen bis das Filtrat kaum mehr gefärbt ist und dann im Trockenschrank bei 120°C getrocknet. Das so erhaltene orange glänzende Pulver wird zweimal mit Methanol extraktiv umkristallisiert, Ausb. 0.30 g (80%) orange Kriställchen, starke rote Festkörperfluoreszenz, Smp. 370-372°C. R_{f} (CHCl₃/Kieselgel)= 0.41. UV (CHCl₃): λₘₐₓ (ε)= 505 (29970), 483 (30309), 354 (3160), 336 (3160), 294 (3300), 264 (29090). Fluoreszenz (CHCl₃): λₘₐₓ (Iᵣₑₗ)= 539 (1), 567 (0.51).

| | | | | |
|---|---|---|---|---|
| C₂₈H₂₁NO₂ (403.5) | Ber. | C 83.35 | H 5.25 | N 3.47 |
| | Gef. | C 83.28 | H 5.38 | N 3.60 |

### Beispiel 13: N-Cycloheptyl-perylen-3,4-dicarboximid

Man mischt 0.30g (0.93 mmol) Perylen-3,4-dicarbonsäureanhydrid mit 0.32 g (2.83 mmol) Cycloheptylamin und 3 g Imidazol und erhitzt die Mischung unter Argon 4 h lang auf 150°C. Das Reaktionsprodukt wird mit Ethanol aus dem Kolben gewaschen, mit 200 ml 10% Salzsäure versetzt und abgesaugt. Der Rückstand wird mit Kaliumcarbonatlösung ausgekocht, abgesaugt und mit heissem dest. Wasser so lange gewaschen, bis das Filtrat farblos abläuft Das so erhaltene Produkt wird im Trockenschrank bei 120°C getrocknet und anschliessend aus Methanol extraktiv umkristallisiert. Ausb. 0.24 g (62%) orange Kristalle mit roter Festkörperfluoreszenz, Smp. 354-355°C, R_{f}
(CHCl₃/Kieselgel)= 0.79. UV (CHCl₃): λₘₐₓ (ε)= 505 (30370), 483 (30967), 353 (3170), 336 (3170), 295 (3460), 264 (29047). Fluoreszenz (CHCl₃): λₘₐₓ (Iᵣₑₗ)= 529 (1), 568 (0.48).

| | | | | |
|---|---|---|---|---|
| C₂₉H₂₃NO₂ (417.5) | Ber. | C 83.43 | H 5.55 | N 3.35 |
| | Gef. | C 82.55 | H 5.62 | N 3.55 |

### Beispiel 13A: N-(2,5-Di-t-butyl-4-nitrophenyl)-perylen-3,4-carboximid

Man mischt 0.20g (0.93 mmol) Perylen-3,4-dicarbonsäureanhydrid mit 0.47g (1.9 mmol) 2,5-Di-t-butyl-4-nitroanilin, 0.20g Zinkacetatdihydrat und 4g Imidazol und erhitzt die Mischung unter Argon 22h lang auf 170°C. Das Produkt wird mit Ethanol aus dem Kolben gewaschen, mit 2N Salzsäure versetzt und dann solange gekocht, bis alles Ethanol verdampft ist. Man saugt ab, wäscht zweimal mit Wasser nach und kocht dann die Mischung mit 10% Kaliumcarbonatlösung. Es wird wiederum heiss abgesaugt und mehrmals mit heissem Wasser nachgewaschen, bis das ablaufende Filtrat nicht mehr gelb gefärbt ist. Der Rückstand wird im Trockenschrank bei 120°C getrocknet und dann mit Toluol über Kieselgel chromatographiert. Die so erhaltene Lösung wird über eine D5-Fritte filtriert, um etwaig anhaftendes Kieselgel zu entfernen. Ausb. 0.09g (26%) rotes Pulver mit roter Festkörperfluoreszenz, Smp. >360°C, R_{f} (CHCl₃/Kieselgel)= 0.31, UV (CHCl₃): λₘₐₓ (ε)= 514 (24450), 489 (25850), 356 (3100), 265 (33790). Fluoreszenz (CHCl₃): λₘₐₓ (Iᵣₑₗ)= 543 (1), 576 (0.45).

| | | | | |
|---|---|---|---|---|
| C₃₆H₃₀N₂O₄ (554.7) | Ber. | C 77.96 | H 5.45 | N 5.05 |
| | Gef. | C 77.78 | H 6.14 | N 4.85 |

### Beispiele 14-18: Nitrierung der Perylen-3,4-dicarboximide

### Beispiel 14: Nitrierung von N-1-Hexylheptyl-perylen-3,4-dicarboximid mit Acetanhydrid/Salpetersäure

Man suspendiert (in Anlehnung an Acta. Chim. Scand. 1983, B37, 65) 120 mg (240 mmol) N-1-Hexylheptyl-perylen-3,4-dicarboximid in 1 ml Acetanhydrid und gibt bei 0°C eine Mischung aus 0.057 ml konz. Salpetersäure in 0.15 ml Acetanhydrid zu. Nach 2 h läßt man die Mischung langsam auf Raumtemperatur erwärmen und rührt weitere 72 h. Man dampft das dunkelrote Produkt im Vakuum ein und chromatographiert den Rückstand mit Chloroform über Kieselgel. Als erste Fraktion erhält man gemäss ¹H-NMR (CDCl₃) eine Mischung aus 60% N-(1-Hexylheptyl)-perylen-3,4-dicarboximid und 40% N-(1-Hexylheptyl)-2-nitro-perylen-3,4-dicarboximid, die jedoch chromatographisch nicht getrennt werden können (R_{f}-Wert 0.85; Chloroform/Kieselgel). Als zweite Fraktion wird gemäss ¹H-NMR (CDCl₃) N-(1-Hexylheptyl)-9-nitro-perylen-3,4-dicarboximid eluiert.

### Beispiel 15: 9-Nitro-N-(1-hexylheptyl)-perylen-3,4-carboximid (10)

Man löst 500 mg (1.00 mmol) N-(1-Hexylheptyl)-perylen-3,4-dicarboximid in 100 ml Dichlormethan, und gibt bei völliger Dunkelheit (Ausschluß von Tageslicht!) 2.9 ml einer Lösung von Stickstoffdioxyd in Dichlormethan (15.7 g NO₂ pro Liter, 1.00 mmol). Man läßt dann die Mischung 17.75 h unter Ausschluß von Licht bei Raumtemperatur stehen und dampft dann das Dichlormethan mit dem Rotationsverdampfer ab. Ein Dünnschichtchromatogramm (Chloroform /Kieselgel) zeigt, daß der erhaltene Rückstand hauptsächlich aus 2 Verbindungen mit den R_{f}-Werten 0.89 und 0.73 besteht. Die Substanz mit dem größeren R_{f}-Wert ist nicht umgesetztes Ausgangsmaterial, von dem durch Säulenchromatographie (Chloroform/Kieselgel) 210 mg (42%) zurückgewonnen werden können. Die 2. Chromatographie-Fraktion wird durch extraktive Umkristallisation aus Methanol gereinigt. Ausb. 300 mg (55%) weinrote Nadeln (Strukturbeleg durch ¹H-NMR-, COSY-, und NOESY-Spektren) Schmp. 184-185°C. R_{f} (Chloroform/Kieselgel)= 0.73. UV (CHCl₃): λₘₐₓ (ε)= 510 nm (34344), 483 nm (30844), 357 nm (breit, 4470). Fluoreszenz (CHCl₃): λₘₐₓ=549 nm.

| | | | | |
|---|---|---|---|---|
| C₃₅H₃₆N₂O₄ (548.7) | Ber. | C 76.62 | H 6.61 | N 5.11 |
| | Gef. | C 76.60 | H 6.58 | N 5.41 |

### Beispiel 16: Nitrierung von N-(2,5-Di-tert-butyl-phenyl)-perylen-3,4-dicarboximid mit N₂O₄ unter Einwirkung von Licht

Man löst (in Anlehnung an Acta. Chim. Scand. 1983, B37, 65) unter der Einwirkung von Tageslicht 250 mg (0.49 mmol) N-(2,5-Di-tert-butyl-phenyl)-perylen-3,4-dicarboximid in 17 ml Dichlormethan und gibt dann 1.5 ml einer Lösung von NO₂ in Dichlormethan zu, die 15.7 g/l enthält (0.5 mmol). Nach einer Reaktionszeit von 17 h bei Raumtemperatur wird das Lösungsmittel mit dem Rotationsverdampfer abgedampft. Ein Dünnschichtchromatogramm (Chloroform/Kieselgel) zeigt, daß der erhaltene Rückstand hauptsächlich aus 3 Farbstoffen mit den R_{f}-Werten 0.27, 0.38 und 0.49 besteht, wobei es sich bei der Verbindung mit dem R_{f}-Wert von 0.38 um das Ausgangsmaterial handelt, wie ein Vergleich mit einer authentischen Probe ergibt. Das Rohprodukt wird mit Chloroform über Kieselgel chromatographiert, wobei man 80 mg (32%) N-(2,5-Di-tert-butylphenyl)-perylen-3,4-dicarboximid zurückgewinnen kann. Durch mehrfache Chromatographie mit Chloroform über Kieselgel und anschließende extraktive Umkristallisation mit Methanol konnte die Verbindung mit einem R_{f}-Wert von 0.49 rein erhalten und durch ¹H-, COSY- und NOESY-NMR-Spektren als 1-Nitro-N-(2,5-di-t-butyl-phenyl)-perylen-3,4-dicarboximid identifiziert werden. Ausb. 20 mg (7%), Smp. 331-335°C (Zers.). R_{f} (Chloroform/Kieselgel)= 0.49. UV (CHCl₃): λₘₐₓ (ε): 514 (25290), 409 (breit, 4350), 356 (breit, 6250).

| | | | | |
|---|---|---|---|---|
| C₃₆H₃₀N₂O₄ (554.6) | Ber. | C 77.96 | H 5.45 | N 5.05 |
| | Gef. | C 77.17 | H 5.64 | N 4.84 |

### Beispiel 17: Nitrierung von N-(1-Hexylheptyl)-perylen-3,4-dicarboximid mit N₂O₄ unter Einwirkung von Licht unter Katalyse von Methansulfonsäure

Man löst (in Anlehnung an Acta. Chim. Scand. 1983, B37, 65) 430 mg (0.85 mmol) N-(2,5-Di-tert-butyl-phenyl)-perylen-3,4-dicarboximid in 25 ml Dichlormethan und gibt zu der roten Lösung 10 µl Methansulfonsäure. Dazu gibt man dann tropfenweise 5 ml einer Lösung von 15.7 g Distickstofftetroxyd im Liter Dichlormethan (0.85 mmol), worauf sie sich sofort nach weinrot umfärbt. Die Lösung läßt man über Nacht s tehen und dampft dann das Dichlor-methan mit dem Rotationsverdampfer ab. Aus einem Dünnschichtchromatogramm (Chloroform/Kieselgel) des weinroten Rückstandes ist die Bildung von 4 Hauptprodukten und einigen Nebenprodukten zu ersehen. Der Rückstand wird mit Chloroform über Kieselgel chromatographiert, wobei alle 4 Hauptprodukte rein erhalten werden können. Nacheinander werden folgende Fraktionen erhalten:
1. Fraktion: 60 mg eines weinroten Pulvers, das durch Extraktion mit Essigester und mehrfache Chromatographie mit Chloroform über Kieselgel weiter gereinigt wird. Ausb. 30 mg (5%) 1,6-Dinitro-N-(1-hexylheptyl)-perylen-3,4-carboximid (durch NMR- und MS- Spektren identifiziert), Schmp. 142-144°C, R_{f} (Chloroform/Kieselgel)= 0.81. UV (CHCl₃): λₘₐₓ= 508, 482. Fluoreszenz (CHCl₃):λₘₐₓ (Iᵣₑₗ)= 541 (1), 573 (sh, 0.72).

| | | | | |
|---|---|---|---|---|
| C₃₅H₃₅N₃O₆ (593.7) | Ber. | C 70.81 | H 5.94 | N 7.08 |
| | Gef. | C 70.88 | H 5.94 | N 6.61 |

2. Fraktion: verunreinigtes 9-Nitro-N-(1-hexylheptyl)-perylen-3,4-carboximid.
3. Fraktion: 0.22g einer weinroten Substanz, die entsprechend den ¹H-NMR-Spektrum aus 81% 9-Nitro-N-(1-hexylheptyl)-perylen-3,4-dicarboximid und 19% 2,5-Dinitro-N-(1-hexylheptyl)-perylen-3,4-dicarboximid besteht. Diese können jedoch weder durch Säulenchromatographie mit Chloroform oder Toluol an Kieselgel oder durch fraktionierte Kristallisation aus Acetonitril oder anderen Lösungsmitteln rein erhalten werden.
4. Fraktion: 130 mg einer dunkelroten Substanz die mit Methanol extraktiv umkristallisiert wird. Man erhält 90 mg (25%) dun-kelrote Kristalle, die durch ein ¹H- und ein NOESY-NMR-Spektrum als 9,10-Dinitro-N-(1-hexylheptyl)-perylen-3,4-dicarboximid identifiziert werden, Schmp. 245-246°C. R_{f} (Chloroform/Kieselgel)= 0.54. UV (CHCl₃): λₘₐₓ (ε)= 506 nm (58015), 474 (40792), 446 (16730).

| | | | | |
|---|---|---|---|---|
| C₃₅H₃₅N₃O₆ (593.7) | Ber. | C 70.81 | H 5.94 | N 7.08 |
| | Gef. | C 70.65 | H 5.78 | N 7.38 |

### Beispiel 18: Nitrierung vonN-(2,5-Di-t-butylphenyl)-perylen-3,4-dicarboximid mit NO₂ unter Lichtausschluss

Man löst (in Anlehnung an Acta. Chim. Scand. 1983, B37, 65) 0.75g (1.47mmol) N-(2,5-Di-t-butylphenyl)-perylen-3,4-dicarboximid in 30 ml abs. Dichlormethan und fügt dann unter Lichtausschluss 6.94 ml (1.48 mmol) einer Lösung dazu, die 19.5 g/l NO₂ in Dichlormethan enthält. Die rote Lösung lässt man über Nacht bei Raumtemperatur stehen und rotiert dann ein. Ein Dünnschichtchromatogramm mit Chloroform über Kieselgel zeigt das Vorhandensein von 3 Verbindungen mit den R_{f}-Werten von 0.43, 0.32 und 0.18. Bei der Verbindung mit dem R_{f}-Wert von 0.32 handelt es sich um das Ausgangsprodukt, wie ein Vergleich mit einer Probe ergibt. Die Verbindung mit dem R_{f}-Wert von 0.43 kann durch wiederholte Flashchromatographie mit Chloroform über Kieselgel (0.04-0.063mm) und anschliessender extraktiver Umkristallisation aus Methanol rein erhalten werden und durch ¹H-, COSY- und NOESY-NMR-Spektren als 1-Nitro-N-(2,5-di-t-butyl-phenyl)-perylen-3,4-dicarboximid identifiziert werden. Ausb. 0.08g (10%) weinrotes Pulver, Smp. >335°C. R_{f} (Chloroform/Kieselgel)= 0.43. UV (CHCl₃): λₘₐₓ (ε): 511 (27690), 409 (breit, 5105), 357 (breit, 7170), 267 (26710).

| | | | | |
|---|---|---|---|---|
| C₃₆H₃₀N₂O₄ (554.6) | Ber. | C 77.96 | H 5.45 | N 5.05 |
| | Gef. | C 77.95 | H 5.33 | N 4.87 |

Die Verbindung mit dem R_{f}-Wert von 0.18 kann durch fraktionierte Flashchromatographie mit Chloroform über Kieselgel (0.04-0.63 mm) und durch zweimalige extraktive Umkristallisation aus Essigester isoliert und durch ¹H-, COSY- und NOESY-NMR-Spektren als 9-Nitro-N-(2,5-di-t-butyl-phenyl)-perylen-3,4-dicarboximid identifiziert werden. Ausb. 0.26g (32%) rotes Pulver, leichte orange Festkörperfluoreszenz. Smp. >365°C, R_{f} (CHCl₃/Kieselgel) 0.18. UV (CHCl₃) λₘₐₓ (ε): 512 (35590), 484 (32104), 358 (4920), 347 (4680), 262 (30924), 255 (29495). Fluoreszenz (CHCl₃) λₘₐₓ (I_{rel.}): 549.

| | | | | |
|---|---|---|---|---|
| C₃₆H₃₀N₂O₄ (554.6) | Ber. | C 77.96 | H 5.45 | N 5.05 |
| | Gef. | C 77.31 | H 5.36 | N 5.16 |

Des weiteren können 0.27g (36%) N-(2,5-Di-t-butylphenyl)-perylen-3,4-dicarboximid zurückgewonnen werden.

### Beispiel 19: 9-Acetamido-N-(1-hexylheptyl)-perylen-3,4-dicarboximid

Man suspendiert 100 mg (0.18 mmol) 9-Nitro-N-(1-hexylheptyl)-perylen-3,4-dicarboximid (**10**) in 15 ml Eisessig, gibt dann 80 mg (0.72 mmol) Eisenstaub zu und kocht die rote Suspension 4.5 h unter Rückfluß, wobei man eine langsame Umfärbung von rot über rotbraun und lila nach dunkellila beobachten kann. Zu der abgekühlten, violetten Lösung gibt man zuerst Wasser und neutralisiert dann mit 10 proz. KOH. Die jetzt rötlichlila gefärbte Suspension wird mit Chloroform dreimal ausgeschüttelt, die vereinigten organischen Phasen werden mit wasserfreiem Natriumsulfat getrocknet und dann einrotiert. Laut Dünnschichtchromatogramm (Chloroform/Kieselgel) besteht das erhaltene lilafarbige Produkt aus **2** Hauptprodukten mit den R_{f}-Werten von 0.02 und 0.43. Dieses wird mit Chloroform über Kieselgel chromatographisch filtriert, bis keine violette Lösung mehr austritt. Das rot gefärbte Kieselgel wird mit Chloroform extrahiert, wobei man nach dem Einrotieren 50 mg rotes Pulver erhält, das anschließend mit Methanol extraktiv umkristallisiert wird. Ausb. an 9-Acetamido-N-(1-hexylheptyl)-perylen-3,4-dicarboximid 30 mg (30%), Schmp. >230°C; ab 172-175°C Braunfärbung. R_{f} (CHCl₃/Eisessig 20:1; Kieselgel)= 0.90. UV (CHCl₃): λₘₐₓ (ε)= 506nm (31589, breit), 356 (2995). Fluoreszenz (CHCl₃): λₘₐₓ= 574 nm (breit).

| | | | |
|---|---|---|---|
| C₃₇H₄₀N₂O₃ (560.7) | Ber. | C 79.25 | H 7.19 |
| | Gef. | C 77.66 | H 7.27 |

### Beispiel 20: 9-Amino-N-(1-hexylheptyl)-perylen-3,4-dicarboximid (17)

Man suspendiert 100 mg (0.18 mmol) 9-Nitro-N-(1-hexylheptyl)-perylen-3,4-dicarboximid und 70 mg Eisenpulver in 30 ml Ethanol und gibt dann langsam 2 ml konz. Salzsäure zu. Die Mischung wird 1 h lang unter Rückfluß erhitzt, wobei man eine Umfärbung nach gelbbraun feststellen kann. Man neutralisiert die Mischung mit KOH, saugt den dunklen Niederschlag ab und trocknet ihn bei 120°C im Trockenschrank. Ein Dünnschichtchromatogramm (Chloroform/Kieselgel) zeigt, daß 2 Substanzen mit den R_{f}-Werten von 0.21 und 0.32 entstanden sind. Man chromatographiert mit Chloroform über Kieselgel, wobei man als erste Fraktion 10 mg eines blau gefärbten Pulvers erhält. Die besteht entsprechend dem NMR- und Massenspektren wahrscheinlich aus zwei Azoverbindungen mit sehr hohen Massenzahlen. Die zweite Fraktion ist 9-Amino-N-(1-hexylheptyl)-perylen-3,4-dicarboximid, Ausb. 80 mg (85%) blaues Pulver, Schmp. 211°C, R_{f}
(Chloroform/Kieselgel)= 0.20. UV (CHCl₃): λₘₐₓ (ε)= 554 nm (27940), 375 (4903), 356 (5412), 277 (29123), 262 (23798). Fluoreszenz (CHCl₃): λₘₐₓ= 642 nm (schwach).

### Beispiel 21: 9-N,N-Dimethylamino-N'-(1-hexylheptyl)-perylen-3,4-carboximid

Man mischt 190 mg (0.37 mmol) 9-Amino-N'-(1-hexylheptyl)-perylen-3,4-carboximid mit 2 g Ameisensäure und gibt dann 40 mg 37 %ige wässrige Formaldehyd-Lösung zu. Diese Suspension wird 18 h auf 75-80°C erhitzt, wobei man eine Umfärbung von Rot nach Blau beobachten kann. Durch die Zugabe von 20 ml 50 %iger KOH-Lösung wird das violette Produkt ausgefällt. Man rührt noch 1 h, saugt dann das Produkt ab, wäscht mit Wasser nach und trocknet im Trockenschrank. Durch wiederholte Chromatographie des Produkts mit Chloroform über Kieselgel können 2 Substanzen mit den R_{f}-Werten 0.92 und 0.51 isoliert werden. Die Substanz mit dem grösseren R_{f}-Wert kann durch
¹H-NMR-Spektroskopie als 9-N,N-Dimethylamino-N'-(1-hexylheptyl)-perylen-3,4-carboximid (70 mg=39 %), die Substanz mit dem kleineren R_{f}-Wert durch Vergleich der Dünnschichtchromatogramme und ¹H-NMR-Spektren als Ausgangsprodukt (40 mg=48 %) identifiziert werden. Ausb.: 70 mg (39 %), Schmp.: 168-169°C
UV (CHCl₃): λₘₐₓ (ε)=542 (26400), 506 sh (21000), 382 (3000), 357 (3200), 269 (25800), 262 (25700). Fluoreszenz (CHCl₃): λₘₐₓ (Iᵣₑₗ)=662.

| | | | | |
|---|---|---|---|---|
| C₃₇H₄₂N₂O₂ (546.8) | Ber. | C 81.28 | H 7.74 | N 5.12 |
| | Gef. | C 80.96 | H 7.67 | N 5.04. |

### Beispiele 22-23-36: Bromierung der Perylen-3,4-dicarboximide

### Beispiel 22: 9-Brom-N-(1-hexylheptyl)-perylen-3,4-carboximid (18c)

Man löst (in Anlehnung an Dyes and Pigments 1991, 16, 19) 410 mg (1.0 mmol) N-(1-Hexylheptyl)-perylen-3,4-dicarboximid (2c) in 70 ml Chlorbenzol, erwärmt auf 40°C und gibt dann schnell eine Lösung von 100µl (4 mmol) Brom in 10 ml Chlorbenzol zu der orangen Lösung, die sich sofort nach Weinrot umfärbt. Man rührt die Lösung 2.5 h lang bei 40-50°C und dampft dann das Chlorbenzol mit dem Rotationsverdampfer ab. Ein Dünnschichtchromatogramm (Toluol/Kieselgel) zeigt die Anwesenheit von 4 Produkten, wobei eines mit dem R_{f}-Wert von 0.77 das Hauptprodukt darstellt. Der Rückstand wird mit Toluol über Kieselgel 8 mal chromatographiert, wobei 30 mg (7%) N-(1-Hexylheptyl)-perylen-3,4-dicarboximid erhalten werden. Als Vorlauf wird ein rein gelbes Produkt isoliert, das entprechend dem Massenspektrum vielfach (2-5) bromierte N-(1-Hexylheptyl)-perylen-3,4-dicarboximide sind. Eine chromatographische Trennung der Verbindungen gelingt nicht. Die Hauptfraktion wird mit Pentan extraktiv umkristallisiert. Ausb. 310 mg (65%) oranges Pulver, das eine starke Festkörperfluoreszenz aufweist, Schmp. 186-187°C. R_{f} (Chloroform/Kieselgel)= 0.77. UV (CHCl₃): λₘₐₓ (ε)= 508 (35675), 484 (33985). Fluoreszenz (CHCl₃) λₘₐₓ (Iᵣₑₗ)= 540 (1), 568 (Sh, 0.49). Die Stellung des Bromatoms kann hierbei durch eine Kombination von ¹H-, NOESY- und COSY-NMR-Spektren aufgeklärt werden.

| | | | | | |
|---|---|---|---|---|---|
| C₃₅H₃₆NO₂Br (582.6) | Ber. | C 72.16 | H 6.23 | N 2.40 | Br 13.72 |
| | Gef. | C 72.13 | H 6.31 | N 2.63 | Br 12.88 |

### Beispiel 23: 9-Brom-N-(2,5-di-tert-butylphenyl)-perylen-3,4-dicarboximid (18b)

Man löst 650 mg (1.28 mmol) N-(2,5-Di-tert-butyl-phenyl)-perylen-3,4-dicarboximid (2b) in 100 ml Chlorbenzol, vermischt die rote Lösung mit 650 mg wasserfreiem Kaliumcarbonat und tropft dann zu der Mischung 0.30 ml Brom in 10 ml Chlorbenzol zu. Man rührt 2 h lang bei 40-50°C, erhöht dann die Temperatur für weitere 5 h auf 50-60°C und dampft dann das Chlorbenzol mit dem Rotationsverdampfer ab, wobei auch noch erhebliche Mengen an Brom mit entfernt werden. Ein Dünnschichtchromatogramm (Toluol/Kieselgel) zeigt kein Ausgangsprodukt mehr im erhaltenen Rückstand an, das nur sehr schwer zu entfernen wäre. Durch mehrmalige säulenchromatographische Trennungen mit Chloroform über Kieselgel können 610 mg oranges Pulver erhalten werden, das mit Essigester extraktiv umkristallisiert wird. Ausb. 570 mg (77%) orangefarbene Nädelchen, die eine leichte Festkörperfluoreszenz aufweisen, Schmp. >320°C. R_{f} (CHCl₃/Kieselgel)= 0.43. UV (CHCl₃): λₘₐₓ (ε)= 511 nm (36058), 486 (35564), 357 (3430). Fluoreszenz (CHCl₃): λₘₐₓ (Iᵣₑₗ)= 542 nm (1), 572 Die Stellung des Bromsubstituenten konnte durch Vergleich mit dem Spektrum von 9-Brom-N-(1-hexylheptyl)-perylen-3,4-dicarboximid und
¹H-Spektren bestimmt werden. Das Substi-tutionsmuster der Vergleichssubstanz wurde durch eine Kombination von COSY- und NOESY-Spektren bestimmt.

| | | | | | |
|---|---|---|---|---|---|
| C₃₆H₃₀NO₂Br (588.6) | Ber. | C 73.47 | H 5.14 | N 2.38 | Br 13.58 |
| | Gef. | C 73.54 | H 5.32 | N 2.40 | Br 13.19 |

(Aus der Vorfraktion läßt sich nach mehrmaliger Säulenchromatographie mit Chloroform über Kieselgel wenig einer weiteren roten Substanz dünnschichtchromatographisch (Chloroform/Kieselgel) rein erhalten. Das Massenspektrum ergibt eine Molekülmasse von 667 mit dem typischen Isotopenmuster von 3 Bromatomen. Die Substanz zeigt jedoch ein ganz ähnliches UV/Vis-Absorptions- und Fluoreszenzspektrum wie 9-Brom-N-(2,5-di-tert-butyl-phenyl)-perylen-3,4-carboximid so daß der Chromophor auf jeden Fall erhalten geblieben sein muß.)

### Beispiel 24: Solvatochromie von 9-Amino-N'-(7-tridecyl)-perylen-3,4-dicarboximid

Die oben erwähnte Verbindung zeigt in ausgewählten Lösungsmitteln geordnet nach steigender Polarität (z.B. gemäss der E_{T}30-Skala) eine ausgeprägte Solvatochromie, indem mit der Erhöhung der Polarität des Lösungsmittels sowohl das UV- als auch das Fluoreszenz-Absorptionsmaximum batochrom verschoben sind. Dies ist beispielsweise aus den entsprechenden Werten in Toluol, im Vergleich zu Ethanol ersichtlich:

| | | |
|---|---|---|
| Toluol | UV λₘₐₓ=550 nm; | Fluoreszenz λₘₐₓ=631 nm |
| Ethanol | UV λₘₐₓ=608 nm; | Fluoreszenz λₘₐₓ=713 nm |

### Beispiel 25: Pigmenteigenschaften von N-(2,5-Di-t-butylphenyl)-perylen-3,4-carboximid bzw. von N-Cyclooctyl-perylen-3,4-dicarboximid

Mit den erwähnten Verbindungen, hergestellt gemäss Beispiel 1 bzw. gemäss Beispiel 7, wird Polyethylenterephthalat (Melinar® 890, ICI) in 0.03% Konzentration gefärbt. Die brilliant orangen Ausfärbungen sind während 5 min bei 300°C wärmestabil; zeigen nach DIN 53775 keine Migration in PVC (24h, 80°C); und weisen eine ausgezeichnete Lichtbeständigkeit nach ISO 105-A02 (5 greyscale nach 500h) auf.

## Patentansprüche

1. Verfahren zur Herstellung von Perylen-3,4-dicarbonsäureimiden der Formel I 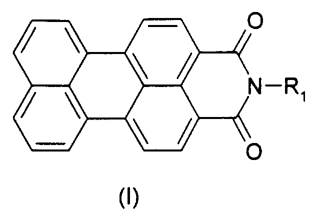 worin R₁ für eine Alkyl-, Aralkyl- oder Cycloalkylgruppe steht oder einen carbocyclischen oder heterocyclischen aromatischen Rest bedeutet, durch Umsetzung des Perylen-3,4,9,10-tetracarbonsäurebisanhydrids mit einem primären Amin R₁-NH₂ bei einer Temperatur von 150-350°C und unter Druck, in Gegenwart von Wasser sowie in Gegenwart eines Zink-, Blei-, Calcium- oder Magnesiumsalzes und eines stickstoffhaltigen Heterocyclus als Base, und wobei das Perylen-Ringsystem nicht substituiert oder einen bis sechs Substituenten aufweisen kann.

2. Verfahren nach Anspruch 1, worin das Salz Bleiacetat, Zinkchlorid oder Zinkacetat ist.

3. Verfahren nach Anspruch 1 oder 2, worin der stickstoffhaltige Heterocyclus Chinolin, Pyridin oder Imidazol ist.

4. Verfahren nach einem der Ansprüche 1 bis 3, worin R₁ ein löslichmachender organischer Rest ist, und für 2,5-Di-tert-butylphenyl, 4-tert.-Butylphenyl, 2,3-Dimethylphenyl, 1-Hexylheptyl, 1-Octylnonyl, 1-Nonyldecyl, Cyclopropyl, Cyclopentyl, Cyclohexyl, Cycloheptyl, Cyclooctyl, Cyclododecyl, Adamantyl oder 4-Carbamoylphenyl steht.

5. Verfahren zur Herstellung von Perylen-3,4-dicarbonsäureimiden der Formel I nach Anspruch 1 durch Umsetzung des Perylen-3,4-dicarbonsäureanhydrids mit einem primären Amin R₁-NH₂ bei einer Temperatur von 150-350°C und unter Druck in Gegenwart eines Zink-, Blei-, Calcium- oder Mangansalzes und eines stickstoffhaltigen Heterocyclus als Base.

## Claims

1. A process for preparing a perylene-3,4-dicarboximide of the formula I 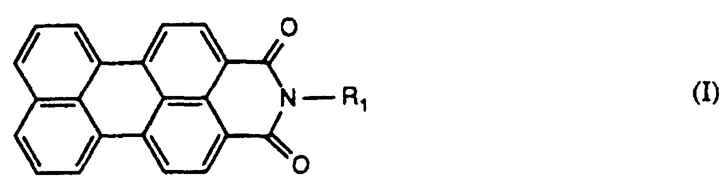 in which R₁ is an alkyl, aralkyl or cycloalkyl group or a carbocyclic or heterocyclic aromatic radical by reacting perylene-3,4,9,10-tetracarboxylic dianhydride with a primary amine R₁-NH₂ at a temperature of 150-350°C and under pressure in the presence of water and in the presence of a zinc salt, lead salt, calcium salt or magnesium salt and of a nitrogen-containing heterocycle as base, the perylene ring system being unsubstituted or having from one to six substituents.

2. A process according to claim 1, in which the salt is lead acetate, zinc chloride or zinc acetate.

3. A process according to claim 1 or 2, in which the nitrogen-containing heterocycle is quinoline, pyridine or imidazole.

4. A process according to one of claims 1 to 3, in which R₁ is a solubilizing organic radical and is 2,5-di-tert-butylphenyl, 4-tert-butylphenyl, 2,3-dimethylphenyl, 1-hexylheptyl, 1-octylnonyl, 1-nonyldecyl, cyclopropyl, cyclopentyl, cyclohexyl, cycloheptyl, cyclooctyl, cyclododecyl, adamantyl or 4-carbamoylphenyl.

5. A process for preparing a perylene-3,4-dicarboximide of the formula I according to claim 1 by reacting perylene-3,4-dicarboxylic anhydride with a primary amine R₁-NH₂ at a temperature of 150-350°C and under pressure in the presence of a zinc salt, lead salt, calcium salt or manganese salt and of a nitrogen-containing heterocycle as base.

## Revendications

1. Procédé pour la préparation de pérylène-3,4-dicarboximides de formule I 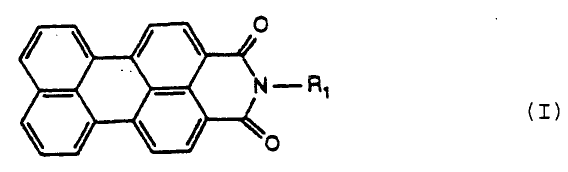 où R₁ représente un groupe alkyle, aralkyle ou cycloalkyle ou un reste aromatique carbocyclique ou hétérocyclique, par réaction du bisanhydride pérylène-3,4,9,10-tétracarboxylique avec une amine primaire R₁-NH₂ à une température de 150 à 350°C et sous pression, en présence d'eau ainsi qu'en présence d'un sel de zinc, de plomb, de calcium ou de magnésium et d'un hétérocycle de azoté comme base, et le système cyclique pérylène n'étant pas substitué ou pouvant porter un à six substituants.

2. Procédé selon la revendication 1, où le sel est l'acétate de plomb, le chlorure de zinc ou l'acétate de zinc.

3. procédé selon la revendication 1 ou 2, où l'hétérocycle azoté est le quinoléine, la pyridine ou l'imidazole.

4. Procédé selon l'une des revendications 1 à 3, où R₁ est un reste organique solubilisant et représente 2,5-di-tert-butylphényle, 4-tert-butylphényle, 2,3-diméthylphényle, 1-hexylheptyle, 1-octylnonyle, 1-nonyldécyle, cyclopropyle, cyclopentyle, cyclohexyle, cycloheptyle, cyclooctyle, cyclododécyle, adamntyle ou 4-carbamoylphényle.

5. Procédé pour la préparation de pérylène-3,4-dicarboximides de formule I selon la revendication 1 par réaction de l'anhydride pérylène-3,4-dicarboxylique avec une amine primaire R₁-NH₂ à une température de 150 à 350°C et sous pression en présence d'un sel de zinc, de plomb, de calcium ou de manganèse et d'un hétérocycle azoté comme base.
